# EUROPEAN PATENT SPECIFICATION

(11) **EP 2 591 780 B1**
(45) Date of publication and mention of the grant of the patent: **13.08.2014**
(21) Application number: 13154095.7
(22) Date of filing: 06.10.2009
(51) Int. Cl.: A61K 31/31, A61K 9/00

(54) **Topical formulations with a tertiary amine oxide**
Topische Formulierungen mit einem tertiären Aminoxid
Formulations topiques avec un oxyde d'amine tertiaire

(30) Priority: 06.10.2008 US 102975 P; 18.08.2009 US 542739
(43) Date of publication of application: 15.05.2013
(62) Divisional of application: 09793308.9
(73) Proprietor: Bausch & Lomb Incorporated, Rochester, NY 14609 (US)
(72) Inventor: Marlowe, Zora Teresa, Rochester, NY New York 14616 (US); Bandyopadhyay, Paramita, Webster, NY New York 14580 (US); Wang, Hongna, South Portland, Maine 04106 (US); Phillips, Eric, Ontario, NY New York 14519 (US)
(74) Representative: Glas, Holger

(56) References cited:
- EP-A1- 0 572 190
- EP-A2- 0 328 355
- WO-A-2008/049042
- JP-A- 2007 106 727
- US-A1- 2004 034 042
- US-A1- 2007 015 710
- US-A1- 2008 194 518

## Description

The present invention relates to formulations that include a pharmaceutical active and a * tertiary amine oxide and sorbic acid as an additional preservative component.

### Background of the Invention

An ophthalmic formulation such as an eye drop formulation that contains a pharmaceutical active typically includes a preservative agent to inhibit growth of bacteria and/or fungi if the formulation becomes contaminated with such organisms. Various preservative agents are known for use in ophthalmic formulations. Such preservative agents should have a broad spectrum of preservative activity and be non-irritating to the eye. Many preservative agents, however, have a tendency to irritate eye tissue, especially if present at relatively high concentrations. Accordingly, ophthalmic formulations that contain relatively small amounts of preservative agent, as described WO 2008/049042 or a preservative agent with a relatively low toxicity profile, are of continued interest.

JP 2007/106727 describes ophthalmical pharmaceutical formulations that contain PEG-type amino oxides as well as tertiary amine oxides. However, JP 2007/106727 discloses merely to use the amine oxides as a cosmetic component and, furthermore, in very high concentrations. Also US 2008/194518 and US 2007/015710 describe ophthalmic formulations that contain tertiary amine oxides in very high amounts.

US 2004/0034042 describes compositions that comprise (a) a xanthine, (b) a buffer and (c) at least one member selected from sorbic acid, EDTA and salts thereof. Also EP 0 328 355 and EP 0 572 190 describe to use sorbic acid in baby wipe products and in aqueous ophthalmic compositions.

U.S. Patent No. 5,840,671 describes a contact lens solution that contains a tertiary amine oxide and an anionic surfactant in the form of a triethanolamine salt. These two cleaning components are present in the solution at a total concentration from 0.1 wt.% to 20 wt.%. Also the weight ratio of the tertiary amine oxide to the anionic surfactant is from 1:4 to 30:1. It is said that the combination of the tertiary amine oxide with the anionic surfactant provide a "higher degree of cleaning effect than any cleaning solution which employs only one of those two kinds of surface active agents." Also, if the total amount of tertiary amine oxide and anionic surfactant is less than 0.1 wt.%, the lens solution "does not exhibit a satisfactory cleaning effect".

### Summary of the Invention

The invention is directed to a topical pharmaceutical formulation comprising 0.5 ppm to 1000 ppm of a tertiary amine oxide of general formula I 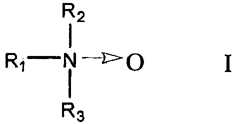 wherein R₁ is a C₈-C₁₈alkyl, and R₂ and R₃ are independently selected from a C₁-C₄alkyl or C₁-C₄hydroxyalkyl, wherein the tertiary amine oxide functions as a preservative or enhances the preservative efficacy of the phamaceutical formulation; a pharmaceutical active agent; and sorbic acid as an additional preservative component.

The invention is also directed to a method to preserve a pharmaceutical formulation.

The method includes adding a tertiary amine oxide of general formula I to the pharmaceutical formulation. The tertiary amine oxide is present from 0.5 ppm to 1000 ppm, and the pharmaceutical formulation is a multi-dose, topical formulation that includes a pharmaceutical active agent. In particular, the tertiary amine oxide is added to an ophthalmic formulation to enhance the preservative efficacy of such formulations.

### Detailed Description of the Invention

As used herein, the term "pharmaceutical active agent" refers to a compound, or a mixture of compounds, that when administered to a subject (human or animal) causes a desired pharmacologic and/or physiologic effect by local and/or systemic action. Accordingly, pharmaceutical formulations comprising a tertiary amine oxide of general formula I have the benefit of being adequately preserved without having a harsh physiological affect such as irritation or discomfort, which is common with many preservative agents. The term "prescription pharmaceutical agent is a pharmaceutical active agent that is included in a formulation in which the formulation requires physician approval (a physician prescription) by the U.S. food and Drug Administration.

The invention is directed to a topical pharmaceutical formulation comprising: a tertiary amine oxide of general formula I 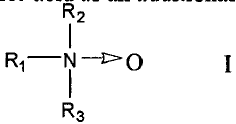 wherein R₁ is a C₈-C₁₈alkyl, and R₂ and R₃ are independently selected from a C₁-C₄alkyl or C₁-C₄hydroxyalkyl; wherein the tertiary amine oxide functions as a preservative or enhances the preservative efficiancy of the pharmaceutical formulation and a pharmaceutical active agent and sorbic acid as an additional preservative component. The invention is also directed to the use of a tertiary amine oxide of general formula I to enhance the preservation of a topical pharmaceutical formulation, particularly an ophthalmic formulation, that includes a pharmaceutical agent and an additional preservative component, 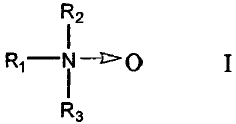 wherein R₁ is a C₈-C₁₈alkyl, and R₂ and R₃ are independently selected from a C₁-C₄alkyl or C₁-C₄hydroxyalkyl, the tertiary amine oxide is present from 0.5 ppm to 1000 ppm and the additional preservative component is sorbic acid.

The tertiary amine oxide of general formula I is present in the formulation from 0.5 ppm to 1000 ppm. In other embodiments, the tertiary amine oxide of general formula I is present in the formulation from 0.5 ppm to 60 ppm.

The presence of the tertiary amine oxide of general formula I in the described topical pharmaceutical formulations demonstrates a preservative "synergy" with another preservative component. The tertiary amine oxide is believed to complement a preservative component in the formulation. In fact, relatively small amounts of tertiary amine oxide are needed to enhance the preservative effectiveness of the formulations, particularly against fungi, e.g., *Fusarium solani* and *Candida albicans.* Typically, formulations that include a tertiary amine oxide of general formula I exhibit a reduction in the cell population by two log orders after 7 days of the five following microorganisms, *Staphylococcus aureus, Pseudomonas aeruginosa, Eschrechia coli, Candida albicans* and *Aspergillus niger,* or will prevent the growth of fungal bioburden by ±0.5 log. See and compare, for example, the stand-alone biocidal data listed in Tables 7 and 8.

As stated, the topical formulations also include an additional preservative component.

The other preservative component that is used in combination with a tertiary amine oxide of general formula I is sorbic acid.

In some ophthalmic formulations such as a multi-dose rewet drop for use with contact lenses or a formulation to alleviate ocular discomfort or dye eye symptoms, the formulations can include one or more demulcents, comfort or cushioning components. Suitable comfort components include, but are not limited to, water soluble natural gums, cellulose-derived polymers and the like and dexpanthenol. Useful natural gums include guar gum, gum tragacanth and hydroxypropyl guar. Useful cellulose-derived comfort components include cellulose-derived polymers, such as hydroxypropyl cellulose, hydroxypropylmethyl cellulose, carboxymethyl cellulose, methyl cellulose, hydroxyethyl cellulose and the like. A very useful comfort component is hydroxypropylmethyl cellulose (HPMC). Some non-cellulose comfort components include propylene glycol or glycerin. The comfort components are typically present in the solution from 0.01% to 1% (w/v).

One preferred comfort agent is hyaluronic acid, which is a linear polysaccharide (long-chain biological polymer) formed by repeating disaccharide units consisting of D-glucuronic acid and N-acetyl-D-glucosamine linked by β(1-3) and β(1-4) glycosidic linkages. Hyaluronic acid is distinguished from the other glycosaminoglycans, as it is free from covalent links to protein and sulphonic groups. Hyaluronic acid is ubiquitous in animals, with the highest concentration found in soft connective tissue. It plays an important role for both mechanical and transport purposes in the body; e.g., it gives elasticity to the joints and rigidity to the vertebrate disks, and it is also an important component of the vitreous body of the eye.

Hyaluronic acid is accepted by the ophthalmic community as a compound that can protect biological tissues or cells from compressive forces. Accordingly, hyaluronic acid has been proposed as one component of a viscoelastic ophthalmic composition for cataract surgery. The viscoelastic properties of hyaluronic acid, that is, hard elastic under static conditions though less viscous under small shear forces enables hyaluronic acid to basically function as a shock absorber for cells and tissues. Hyaluronic acid also has a relatively large capacity to absorb and hold water. The stated properties of hyaluronic acid are dependent on the molecular weight, the solution concentration, and physiological pH. At low concentrations, the individual chains entangle and form a continuous network in solution, which gives the system interesting properties, such as pronounced viscoelasticity and pseudoplasticity that is unique for a water-soluble polymer at low concentration.

Another comfort agent of specific interest is alginate. Alginate is an anionic biopolymer produced by a variety of microorganisms and marine algae. Alginate is a polysaccharide that comprises β-D-mannuronic acid units and α-L-guluronic acid units. Some alginate polymers are block copolymers with blocks of the guluronic acid (or salt) units alternating with blocks of the mannuronic acid (or salt) units as depicted in-part below.

Some alginate molecules have single units of guluronic acid (or salt) alternating with single units of mannuronic acid (or salt). The ratio and distribution of the mannuronic and guluronic unit, along with the average molecular weight, affect the physical and chemical properties of the copolymer. See Haug, A. et al., Acta Chem. Scand., 183-90 (1966). Alginate polymers have viscoelastic rheological properties and other properties that make it suitable for some medical applications. See Klock, G. et al., "Biocompatibility of mannuronic acid-rich alginates," Biomaterials, Vol. 18, No. 10, 707-13 (1997). The use of alginate as a thickener for topical ophthalmic use is disclosed in U.S. Patent No. 6,528,465 and U.S. Patent Application Publication 2003/0232089. In U.S. Patent No. 5,776,445, alginate is used as a drug delivery agent that is topically applied to the eye. U.S. Patent Publication No. 2003/0232089 teaches a dry-eye formulation that contains two polymer ingredients including alginate.

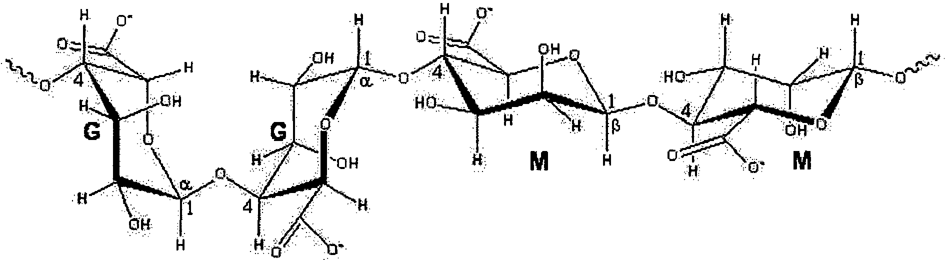

The alginate used in the formulations will typically have a number average molecular weight from about 20 kDa to 2000 kDa, or from about 100 kDa to about 1000 kDa, for example about 325 kDa. The concentration of alginate is from about 0.01 wt.% to about 2.0 wt.%. More, typically, the concentration of alginate is from about 0.1 wt.% to about 0.5 wt.%.

In some instances, the topical formulation will include a prescription pharmaceutical agent. Also, as mentioned, the topical formulation can be an ophthalmic formulation prescribed by or recommended by a physician, or a health care provider, e.g., an O.D., for the treatment of an ocular condition or an ocular disease. Likewise, the ophthalmic formulation can also include a prescription pharmaceutical agent.

Exemplary reference ophthalmic formulations for the treatment of dry eye are provided in Tables 1 to 5. Each component is listed as % w/w except as noted and the alkylamine oxide is myristamine oxide, lauramine oxide or any mixture thereof. Additional information on dry eye formulations can be found in U.S. patent application serial no. 11/842,394, filed August 21, 2007, now U.S. Publication No. 2008/0057022.

**Table 1.**

| **Component** | **% w/w** |
|---|---|
| Carbopol® 980NF | 0.02 to 0.2 |
| glycerin | 0.01 to 0.5 |
| alkylamine oxide | 0.001 to 0.1 |
| PHMB (ppm) | 0.3 to 1.1 |
| sorbitol | 0.5 to 5.0 |
| purified water | q.s. to 100% |

Other reference ophthalmic formulations of a sterile, buffered, hypotonic solution intended for use as an artificial tear and lubricant for providing soothing therapy to dry irritated eyes are listed in Tables 2 to 5. For example, the solutions of Table 2 are non-blurring, low viscosity solutions that contains propylene glycol and glycerin as demulcents, which are believed to lubricate or cushion the corneal epithelium. The solution also contains alginate, a hydrocolloid, which is believed to interact with the mucin layer in the tear film and maintain a moist ocular environment for an extended period of time. The alginate is also believed to stabilize the tear film and provide long term relief to dry eyes.

**Table 2.**

| **Component** | **% w/w** |
|---|---|
| Protanal LF200M alginate | 0.05 to 0.5 |
| glycerin | 0.1 to 1.0 |
| propylene glycol | 0.1 to 1.0 |
| alkylamine oxide | 0.001 to 0.1 |
| PHMB (ppm) | 0.3 to 0.8 |
| sodium borate | 0.01 to 0.04 |
| boric acid | 0.2 to 0.8 |
| Dequest®2016 | 0.02 to 1.2 |
| purified water, USP | Q.S. to 100% |

**Table 3.**

| **Component** | **% w/w** |
|---|---|
| hydroxyethyl cellulose or hydroxypropyl guar | 0.2 to 2.0 |
| propylene glycol | 2.0 to 20 |
| alkylamine oxide | 0.001 to 0.1 |
| Polyquaternium-1 (ppm) | 1 to 5 |
| EDTA | 0.02 to 0.25 |
| purified water, USP | Q.S. to 100% |

**Table 4.**

| **Component** | **% w/w** |
|---|---|
| mineral oil | 3.0 to 7.0 |
| phosphate buffer ^{a} | 0.1 to 0.5 |
| alkylamine oxide | 0.001 to 0.1 |
| PHMB (ppm) | 0.3 to 0.8 |
| EDTA | 0.005 to 0.02 |
| surfactant ^{b} | 0.2 to 1.0 |
| NaCl | 0.3 to 1.0 |
| purified water, USP | Q.S. to 100% |

| | |
|---|---|
| a - A mixture of NaHPO₄ and Na₂PO₄. b - A mixture of polysorbate 80 and PEG-40-octylphenyether | |

**Table 5.**

| **Component** | **% w/w** |
|---|---|
| hyaluronate | 0.05 to 0.25 |
| phosphate buffer ^{a} | 0.1 to 0.5 |
| alkylamine oxide | 0.001 to 0.1 |
| glycerin | 0.3 to 1.2 |
| EDTA | 0.005 to 0.02 |
| NaCl | 0.3 to 1.0 |
| purified water, USP | Q.S. to 100% |

| | |
|---|---|
| a - A mixture of NaHPO₄ and Na₂PO₄. | |

An reference exemplary set of ophthalmic formulations with amino alcohol/zinc salt preservative system are provided in Table 6.

**Table 6.**

| **Component** | **% w/w** |
|---|---|
| hydroxypropyl guar | 0.1 to 0.6 |
| borate acid | 0.2 to 0.7 |
| alkylamine oxide | 0.001 to 0.1 |
| ZnCl | 0.3 to 1.2 |
| AMP | 0.05 to 0.5 |
| propylene glycol | 0.5 to 2.0 |
| purified water, USP | Q.S. to 100% |

### 1. Use of one or more alkyl amineoxides in a a topical pharmaceutical formulation.

The pharmaceutical active agent can be any compound that is used to treat any one disease or any one medical condition. Accordingly, the pharmaceutical active agent can be selected from any one class of compounds, for example, anti-inflammatory agents, anti-infective agents (including antibacterial, antifungal, antiviral, antiprotozoal agents), anti-allergic agents, antiproliferative agents, anti-angiogenic agents, anti-oxidants, antihypertensive agents, neuroprotective agents, cell receptor agonists, cell receptor antagonists, immunomodulating agents, immunosuppressive agents, IOP lowering agents, beta adrenoceptor antagonists, alpha-2 adrenoceptor agonists, carbonic anhydrase inhibitors, cholinergic agonists, prostaglandins and prostaglandin receptor agonists, angiotensin converting enzyme ("ACE") inhibitors, AMPA receptor antagonists, NMDA antagonists, angiotensin receptor antagonists, somatostatin agonists, mast cell degranulation inhibitors, alpha-adrenergic receptor blockers, alpha-2 adrenoceptor antagonists, thromboxane A2 mimetics, protein kinase inhibitors, prostaglandin F derivatives, prostaglandin-2 alpha antagonists, cyclooxygenase-2 inhibitors and muscarinic agents.

Of particular interest are pharmaceutical active agents that are known to treat an ocular disease or disorder including, but are not limited to, a posterior-segment disease or disorder. In certain embodiments, such ocular disease or disorder is selected from the group consisting of diabetic retinopathy, diabetic macular edema, cystoid macular edema, age macular degeneration (including the wet and dry form), optic neuritis, retinitis, chorioretinitis, intermediate and posterior uveitis and choroidal neovascuralization.

Glaucoma is a group of diseases that are characterized by the death of retinal ganglion cells ("RGCs"), specific visual field loss, and optic nerve atrophy. Glaucoma is the third leading cause of blindness worldwide. An intraocular pressure ("IOP") that is high compared to the population mean is a risk factor for the development of glaucoma. However, many individuals with high IOP do not have glaucomatous loss of vision. Conversely, there are glaucoma patients with normal IOP. Therefore, continued efforts have been devoted to elucidate the pathogenic mechanisms of glaucomatous optic nerve degeneration.

It has been postulated that optic nerve fibers are compressed by high IOP, leading to an effective physiological axotomy and problems with axonal transport. High IOP also results in compression of blood vessels supplying the optic nerve heads ("ONHs"), leading to the progressive death of RGCs. See; e.g., M. Rudzinski and H.U. Saragovi, Curr. Med. Chem.-Central Nervous System Agents, Vol. 5, 43 (2005).

In one embodiment, the anti-glaucoma pharmaceutical agent is of general formula II 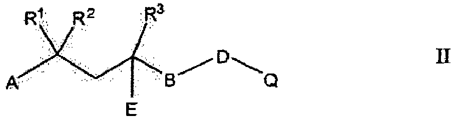 wherein A and Q are independently selected from the group consisting of aryl and heteroaryl groups substituted with at least a halogen atom, cyano group, hydroxy group, or C₁-C₁₀ alkoxy group; R¹, R², and R³ are independently selected from the group consisting of unsubstituted and substituted C₁-C₅ alkyl groups; B is a C₁-C₅ alkylene group; D is the NH- or -NR'- group, wherein R' is a C₁-C₅ alkyl group; and E is the hydroxy group.

Exemplary, pharmaceutical agents of general formula II include A as a dihydrobenzofuranyl group substituted with a fluorine atom; Q as a quinolinyl or isoquinolinyl group substituted with a methyl group; R¹ and R² are independently selected from the group consisting of unsubstituted and substituted C₁-C₅ alkyl groups; B is a C₁-C₃ alkylene group; D is the -NH- group; E is a hydroxy group; and R³ is a trifluoromethyl group.

Exemplary compounds include a glucocorticoid receptor agonist having Formulae III or IV, as disclosed in US Patent Application Publication 2006/0116396. 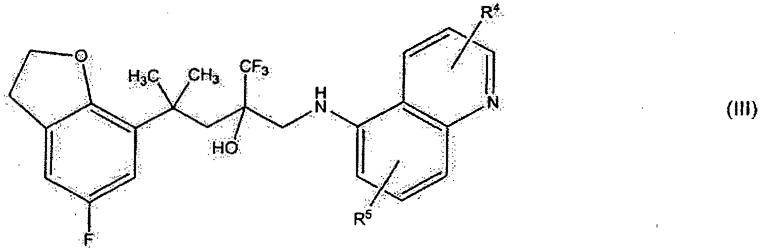 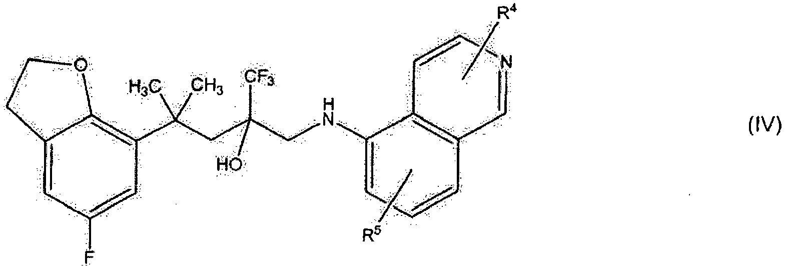 wherein R⁴ and R⁵ are independently selected from the group consisting of hydrogen, halogen, cyano, hydroxy, C₁-C₁₀ (alternatively, C₁-C₅ or C₁-C₃) alkoxy groups, unsubstituted C₁-C₁₀ (alternatively, C₁-C₅ or C₁-C₃) linear or branched alkyl groups, substituted C₁-C₁₀ (alternatively, C₁-C₅ or C₁-C₃) linear or branched alkyl groups, unsubstituted C₃-C₁₀ (alternatively, C₃-C₆ or C₃-C₅) cyclic alkyl groups, and substituted C₃-C₁₀ (alternatively, C₃-C₆ or C₃-C₅) cyclic alkyl groups.

Another embodiment includes ocular formulations prescribed by or recommended by a physician, or a health care provider, to treat an ocular allergic conditions. Allergy is characterized by a local or systemic inflammatory response to allergens. Allergic conjunctivitis is a disorder that is characterized by the clinical signs and symptoms of eye itching, redness, tearing, and swelling. An estimated 20% of the population in the United States suffer from inflammation of the eye. The signs and symptoms of allergic conjunctivitis can significantly impact the quality of life of patients, from social interactions, productivity at work and school, to the ability to perform visual tasks such as working on a computer or reading.

Currently, available pharmaceutical treatments for inflammation of the eye or symptoms of inflammation of the eye include (1) antihistamines, (2) drugs that block the release of histamine and other substances from the mast cell (e.g., mast cell stabilizers), (3) drugs with multiple modes of action (e.g. antihistamine/mast cell stabilizing agents), and (4) drugs that can actively constrict blood vessels thus reducing redness and swelling (e.g., vasoconstrictors). Additionally, artificial tears have been used to wash the eye of allergens.

The desirability of a particular treatment for inflammation of the eye can be measured against the following factors (1) efficacy at onset of action, (2) duration of action, (3) efficacy at controlling signs and symptoms of allergic conjunctivitis, and (4) comfort of the drop when instilled in the eye.

In still another aspect, the formulation comprising: (a) ketotifen or a salt thereof in a concentration of from a 0.001% to 0.2% (weight/volume or "w/v"); (b) naphazoline or a salt thereof in a concentration of from 0.001% to 0.2% (w/v); and (c) water.

Ketotifen or any ophthalmically acceptable ketotifen salt may be used in the method herein described, although ketotifen fumarate is preferred. Ketotifen fumarate is represented by the following formula:

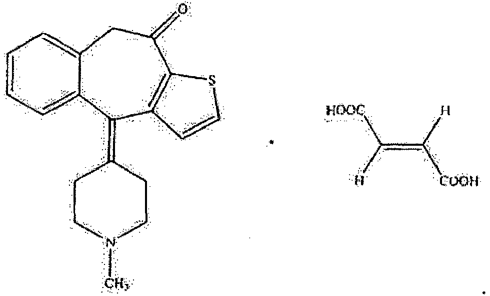

Ketotifen or a ketotifen salt is present in a formulation in a concentration from 0.001% to 0.2% (or alternatively, from 0.001% to 0.1%). In one embodiment, ketotifen or a ketotifen salt is present in a concentration from 0.01% to 0.05%; preferably, from 0.01% to 0.04%; more preferably, from 0.02% to 0.03%. Concentrations of ketotifen salts yielding such concentrations of ketotifen may be readily calculated; for example, using ketotifen fumarate in a concentration of 0.0345% provides a concentration of ketotifen in the formulation of 0.025%.

Another embodiment is directed to an ocular formulation that includes an anti-redness agent, which may relieve redness in the eye. The preferred anti-redness agent is naphazoline or an ophthalmically acceptable salt thereof such as, for example, naphazoline hydrochloride. Other anti-redness agents that may be used include, but are not limited to, tetrahydrozoline, ephedrine, phenylephrine, oxymetazoline, xylometazoline, pseudoephedrine, tramazoline, other vasoconstrictors, combinations thereof, as well as ophthalmically acceptable salts thereof (e.g., tetrahydrozoline hydrochloride).

Naphazoline hydrochloride is represented by the following formula:

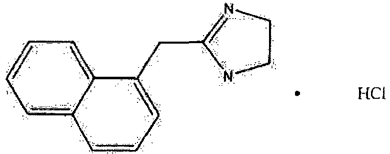

Naphazoline or a naphazoline salt may be present in a composition produced a method of the present invention in a concentration from 0.001% to 0.2% (or alternatively, from 0.001% to 0.1%). In one embodiment, naphazoline or a naphazoline salt is present in a composition at a concentration from 0.01% to 0.1%; preferably, from 0.01% to 0.07%; more preferably, from 0.02% to 0.06%. Concentrations of a naphazoline salt yielding such concentrations of naphazoline base may be readily calculated; for example, using naphazoline hydrochloride in a concentration of about 0.025% provides a concentration of naphazoline base in the formulation of 0.021%. Additional information on formulations containing ketotifen, naphazoline or a corresponding pharmaceutically salt of each thereof can be found in U.S. patent application serial no. 10/972,571,filed October 25, 2005.

Ophthalmic compositions will typically include tonicity agents, e.g., salts such as NaCl, to approximate the osmotic pressure of normal lachrymal fluids, which, as stated in U.S. Patent No. 6,274,626, is equivalent to a 2.5% solution of glycerol. Osmotic pressure, measured as osmolality, is generally about 180 to 420 mOsm/kg for conventional ophthalmic formulations.

In particular, ophthalmic formulations for the treatment of dry-eye will at times have a measured osmolality from 180 to 280 mOsm/kg.

In some embodiments, however, the pharmaceutical formulation may be formulated to osmolality in the range from about 400 to about 875 mOsm/kg, for some desired purposes. For example, co-assigned U.S. Patent Application No. 2006/0148899, incorporated herein by reference in its entirety, provides for ophthalmic formulations having osmolality from 400 to 875 mOsm/kg, which have been found still to provide comfort to a user.

Another embodiment is directed to a pharmaceutical formulation prescribed by or recommended by a physician, or a health care provider, to treat a dermatological condition or a dermatological disease. For example, it is known that compounds of the FK506 class can be formulated into stable emulsions. Emulsions, since they contain an aqueous phase, are much less occlusive than oil-based compositions and hence are better tolerated in many situations. Accordingly, in one embodiment a topical formulation, in the form of an emulsion, comprises a compound of the FK506 class, a physiologically acceptable alkanediol, ether diol or diether alcohol containing up to 8 carbon atoms as solvent for the compound of the FK506 class and one or more alkyl amineoxides as a preservative agent. A compound of the "FK506 class" is a compound which has the basic structure as FK506 and which has at least one of the biological properties of FK506 (e.g., immunosuppressant properties). The compound may be in free base form or pharmaceutically acceptable, acid addition, salt form. A preferred compound of the FK 506 class is disclosed in EP 427 680, e.g. Example 66a (also called 33-epi-chloro-33-desoxyascomycin).

### Reference Examples

The following borate buffered formulations were prepared and are reported in Tables 7 and 8. Each formulation includes 0.6 wt.% boric acid and 0.1 wt.% sodium borate along with the other components listed. All the formulations are reference examples except example 15.

**Table 7.**

| **Reference Example** | **C1** | **1** | **2** | **3** | **C2** | **4** |
|---|---|---|---|---|---|---|
| NaCl | 0.4 | 0.4 | 0.4 | 0.4 | 0.5 | 0.5 |
| PHMB (ppm) | 1 | 1 | -- | 1 | 0.5 | 0.5 |
| lauramine oxide (ppm) | -- | 300 | 300 | 150 | -- | 100 |
| Biocidal data at 4 hours with 10% organic soil | | | | | | |
| S. aureus | 2.7 | 4.7 | >4.6 | 4.7 | 2.0 | 4.6 |
| P. aeruginosa | -- | >4.7 | 1.1 | >4.7 | 2.4 | >4.7 |
| S. marcescens | -- | >4.7 | 1.2 | >4.7 | 2.4 | >4.7 |
| C. albicans | 2.5 | >4.7 | 4.1 | >4.6 | 1.1 | 4.6 |
| F. solani | 2.1 | 4.3 | >4.3 | 4.3 | 1.5 | 4.5 |

**Table 8.**

| **Reference Example** | **C3** | **5** | **C4** | **6** | **7** | **8** |
|---|---|---|---|---|---|---|
| NaCl | 0.5 | 0.5 | 0.4 | 0.4 | 0.5 | 0.4 |
| PHMB | -- | -- | 1.1 | 1.1 | -- | -- |
| Polylysine (ppm) | 10 | 10 | -- | -- | -- | -- |
| propylene glycol alginate | -- | -- | 0.005 | 0.005 | -- | -- |
| lauramine oxide. | -- | 100 | -- | 300 | 100 | 150 |

| **Biocidal data at 4 hours with 10% organic soil** | | | | | | |
|---|---|---|---|---|---|---|
| S. aureus | 2.5 | 4.8 | 2.6 | 4.7 | 3.1 | >4.6 |
| P. aeruginosa | >4.7 | >4.7 | >4.7 | >4.7 | 1.2 | 1.1 |
| S. marcescens | 3.3 | 4.7 | 4.3 | >4.7 | 1.2 | 1.2 |
| C. albicans | 0.4 | 4.4 | 0.7 | >4.7 | 4.6 | 4.6 |
| F. solani | 0.6 | 4.3 | 1.4 | 4.3 | >4.5 | >4.3 |

### Reference Example 9.

A pharmaceutical formulation is prepared in accordance with the components listed in Table 9.

**Table 9.**

| **Component** | **% w/w** |
|---|---|
| betaxolol HCl | 0.5 |
| NaCl | 0.1 |
| Polyquaternium-1 | 0.001 |
| myristamine oxide | 0.005 |
| EDTA | 1.0 |
| purified water, USP | 98 |

### Reference Examples 10 and 11 and C5

The following borate buffered formulations were prepared and are reported in Table 10. Each formulation includes 0.5 wt.% boric acid and 0.014 wt.% sodium borate along with the other components listed. The preservative efficacy data at 14 and 28 days is also reported in Table 10 for each of the Example formulations. The C5, C6, 10A and 11A data is the initial PE data, and the 10B and 11B data is the PE data following storage in poly(ethylene terephalate) (PET) bottles for four months at 40 °C. As indicated the preservative effectiveness of the formulations is maintained upon storage under relatively high temperature conditions.

More importantly, however, is the significant degree of preservative efficacy observed in the sodium alginate solutions. In prior attempts to develop ophthalmic formulations that include both sodium alginate and PHMB, Applicants and others at Bausch & Lomb observed a dramatic fall off in preservative effectiveness of the formulations. One suspected some form of complexation between the cationic PHMB and the anionic alginate that would essentially tie-up the PHMB. The presence of the alkyl amine oxide somehow mitigates this complexation, and quite surprisingly, the preservative efficacy of the formulations are maintained even after four months at 40 °C.

**Table 10.**

| **Example** | **C5** | **10** | | **11** | | **C6** |
|---|---|---|---|---|---|---|
| Na alginate ^{a} | 0.25 | 0.25 | | 0.25 | | 0.25 |
| PHMB (ppm) | 1.0 | 1 | | 2.0 | | 2.0 |
| lauramine oxide (ppm) | -- | 100 | | 100 | | -- |
| glycerin | 0.6 | 0.6 | | 0.6 | | 0.6 |
| propylene glycol | 0.6 | 0.6 | | 0.6 | | 0.6 |
| Dequest®2016 | 0.05 | 0.05 | | 0.05 | | 0.05 |

| **USP PE Test (days)** | **C5** | **10A** | **10B** | **11A** | **11B** | **C6** |
|---|---|---|---|---|---|---|
| S. Aureus (14) | 3.7 | >4.7 | >4.8 | >4.7 | >4.8 | >4.7 |
| (28) | >4.7 | >4.7 | >4.8 | >4.7 | >4.8 | >4.7 |
| P. Aeruginosa (14) | 1.6 | 4.7 | 4.5 | >4.7 | >4.8 | 1.6 |
| (28) | 0.7 | >4.7 | >4.8 | >4.7 | >4.8 | 0.8 |
| E. Coli (14) | 1.6 | 3.3 | 2.2 | >4.7 | >4.8 | 2.4 |
| (28) | 0.8 | >4.7 | >4.8 | >4.7 | >4.8 | 3.6 |
| C. Albicans (14) | 0.7 | >48 | >5.0 | >4.8 | >5.0 | 0.8 |
| (28) | 2.5 | >4.8 | >5.0 | >4.8 | >5.0 | 3.0 |
| A. Niger (14) | 1.2 | >4.7 | 4.7 | 4.1 | >4.7 | 1.2 |
| (28) | 1.2 | >4.7 | >4.7 | >4.7 | >4.7 | 1.1 |

| | | | | | | |
|---|---|---|---|---|---|---|
| a Protanal LF200M | | | | | | |

### Reference Example 12.

An ophthalmic comfort (dry eye) formulation is prepared in accordance with the components listed in Table 11. The preservative efficacy data at 14 and 28 days is also reported in Table 11 for the formulation. The 12A is the initial PE data, and the 12B data is the PE data following storage in poly(ethylene teraphthalate) (PET) bottles for four months at 40 °C. As indicated the preservative effectiveness of the formulation is maintained upon storage under relatively high temperature conditions.

**Table 11. Reference Example 12**

| | | |
|---|---|---|
| boric acid | 0.5 | |
| Na hyaluronate | 0.15 | |
| polyvinylpyrrolidone | 0.1 | |
| PHMB (ppm) | 1.3 | |
| lauramine oxide (ppm) | 100 | |
| glycerin | 0.6 | |
| propylene glycol | 0.6 | |
| Dequest®2016 | 0.10 | |

| **USP PE Test (days)** | **12A** | **12B** |
|---|---|---|
| S. Aureus (14) | >4.8 | >4.8 |
| (28) | >4.8 | >4.8 |
| P. Aeruginosa (14) | >4.8 | >4.8 |
| (28) | >4.8 | >4.8 |
| E. Coli (14) | >4.7 | >4.8 |
| (28) | >4.7 | >4.8 |
| C. Albicans (14) | >4.9 | >5.0 |
| (28) | >4.9 | >5.0 |
| A. Niger (14) | 4.1 | >4.7 |
| (28) | >4.7 | >4.7 |

### Reference Examples 13 14, 16 and C6 and Example 15.

Aqueous ophthalmic formulations, which can be used as a contact lens rewet eye drop formulation, are provided in Table 12.

**Table 12.**

| **Component (wt.%)** | **13** | **14** | **15** | **16** | **C6** |
|---|---|---|---|---|---|
| sodium hyaluronate | 0.1 | 0.1 | 0.1 | 0.1 | 0.1 |
| sodium chloride | 0.42 | 0.42 | 0.42 | 0.42 | 0.42 |
| potassium chloride | 0.14 | 0.14 | 0.14 | 0.14 | 0.14 |
| boric acid | 0.6 | 0.6 | 0.6 | 0.6 | 0.6 |
| sodium borate | 0.035 | 0.035 | 0.035 | 0.035 | 0.035 |
| calcium chloride | 0.006 | 0.006 | 0.006 | 0.006 | 0.006 |
| magnesium chloride | 0.006 | 0.006 | 0.006 | 0.006 | 0.006 |
| lauramine oxide (ppm) | 50 | 50 | 50 | 50 | - |
| stabilized oxy-chloride (purite) | 0.003 | -- | -- | -- | 0.003 |
| PHMB (ppm) | -- | 0.8 | -- | | -- |
| sorbic acid | -- | - | 0.005 | | -- |
| Na perborate (ppm) | -- | - | -- | 400 | -- |

### Reference Examples 17 to 20.

Aqueous ophthalmic formulations, which can be used as a contact lens rewet eye drop formulation, are provided in Table 13.

**Table 13.**

| **Component (wt.%)** | **17** | **18** | **19** | **C7** |
|---|---|---|---|---|
| hydroxypropyl guar gum | 0.15 | 0.1 | 0.15 | 0.15 |
| HPMC E4M | 0.3 | 0.2 | -- | 0.3 |
| hyaluronic acid | -- | 0.1 | 0.2 | -- |
| boric acid | 0.8 | 0.8 | 0.8 | 0.8 |
| sodium borate | 0.035 | 0.035 | 0.035 | 0.035 |
| propylene glycol | 0.8 | 0.8 | 0.8 | 0.8 |
| glycerol | 0.3 | 0.3 | 0.03 | 0.3 |
| lauramine oxide (ppm) | 50 | 50 | 50 | -- |
| polyquaternium-1 (ppm) | 1 | 1 | 1 | 1 |

### Reference Example 20.

An ophthalmic comfort (dry eye) formulation is prepared in accordance with the components listed in Table 14. The preservative efficacy data at 14 and 28 days is also reported in Table 14 for the formulation.

**Table 14.**

| **Example** | **C8** | **20** |
|---|---|---|
| boric acid | 0.5 | 0.5 |
| Na hyaluronate | 0.2 | 0.2 |
| PVP K90 | 0.1 | 0.1 |
| Na perborate (ppm) | 400 | 400 |
| lauramine oxide (ppm) | -- | 100 |
| glycerin | 0.6 | 0.6 |
| propylene glycol | 0.6 | 0.6 |
| EDTA | 0.02 | 0.2 |

| **USP PE Test (days)** | | |
|---|---|---|
| S. Aureus (14) | >4.8 | >4.8 |
| (28) | >4.8 | >4.8 |
| P. Aeruginosa (14) | >4.8 | >4.8 |
| (28) | >4.8 | >4.8 |
| E. Coli (14) | >4.8 | >4.8 |
| (28) | >4.8 | >4.8 |
| C. Albicans (14) | 3.5 | >5.0 |
| (28) | >5.0 | >5.0 |
| A. Niger (14) | 0.8 | >4.7 |
| (28) | 1.0 | >4.7 |

In view of the above, some aspects of the invention relate to:
1. A topical pharmaceutical formulation comprising: 0.5 ppm to 1000 ppm of a tertiary amine oxide of general formula I 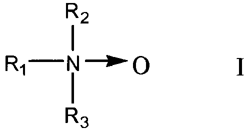 wherein R₁ is a C₈-C₁₈alkyl, and R₂ and R₃ are independently selected from a
   C₁-C₄alkyl or C₁-C₄hydroxyalkyl; and a pharmaceutical active agent, wherein the tertiary amine oxide functions as a preservative or enhances the preservative efficacy of the pharmaceutical formulation.
2. The formulation of aspect 1 further comprising an additional preservative component.
3. The formulation of aspect 2 wherein the preservative component is a cationic, preservative component selected from the group consisting of quaternary ammonium compounds and their polymers and biguanides and their polymers.
4. The formulation of any one of aspects 1 to 3 wherein the tertiary amine oxide is present at a concentration from 0.5 ppm to 300 ppm.
5. The formulation of any one of aspects 1 to 4 wherein the tertiary amine oxide includes myristamine oxide or lauramine oxide.
6. The formulation of any one of aspects 1 to 5 further comprising a comfort component selected from the group consisting of hydroxypropyl guar, hyaluronic acid, alginate, dexpanthenol and hydroxypropylmethyl cellulose.
7. The formulation of aspect 3 wherein the cationic, preservative component is selected from poly(hexamethylene biguanide), which is present in the formulation from 0.1 ppm to 1.3 ppm, alexidine, which is present in the formulation from 0.5 ppm to 5 ppm, or α-[4-tris(2-hydroxyethyl)-ammonium chloride-2-butenyl]poly[1-dimethyl ammonium chloride-2-butenyl]-co-tris(2-hydroxyethyl) ammonium chloride (polyquaternium-1), which is present in the formulation from 0.5 ppm to 3 ppm, or polyquaternium-42.
8. The formulation of any one of aspects 2 and 4 to 6 wherein the preservative component is selected from the group consisting of polylysine, diglycine, sorbic acid, polypeptide and hydrogen peroxide or a stabilized form of hydrogen peroxide.
9. The formulation of any one of aspects 1 to 8 wherein the tertiary amine oxide is present from 0.5 ppm to 60 ppm.
10. The formulation of any one of aspects 1 to 9 wherein the pharmaceutical agent is a prescription pharmaceutical agent.
11. The formulation of any one of aspects 4 to 10 wherein the formulation is a preserved ophthalmic composition having an osmolality of 225 mOsm/kg to 400 mOsm/kg.
12. The use of a tertiary amine oxide of general formula I to contribute to the preservation of a topical pharmaceutical formulation that includes a pharmaceutical agent 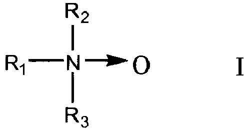 wherein R₁ is a C₈-C₁₈alkyl, and R₂ and R₃ are independently selected from a
   C₁-C₄alkyl or C₁-C₄hydroxyalkyl, the tertiary amine oxide is present from 0.5 ppm to 1000 ppm.
13. The use of aspect 12 wherein the tertiary amine oxide is myristamine oxide or lauramine oxide, which is present at a concentration from 0.5 ppm to 300 ppm.
14. The use of aspects 12 or 13 wherein the tertiary amine oxide and a cationic, preservative component selected from the group consisting of quaternary ammonium compounds and their polymers and biguanides and their polymers is used to preserve the pharmaceutical formulation.
15. The use of aspects 12 or 13 wherein the tertiary amine oxide and a preservative component selected from the group consisting polylysine, diglycine, sorbic acid, polypeptide and hydrogen peroxide or a stabilized form of hydrogen peroxide is used to preserve the pharmaceutical formulation.
16. The use of any one of aspects 12 to 15 wherein the pharmaceutical active agent present in the pharmaceutical formulation provides for the treatment of an ocular disease or disorder.

## Claims

1. A topical pharmaceutical formulation comprising:
0.5 ppm to 1000 ppm of a tertiary amine oxide of general formula I 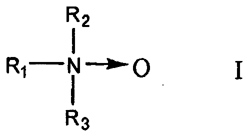
wherein R₁ is a C₈-C₁₈alkyl, and R₂ and R₃ are independently selected from a C₁-C₄alkyl or C₁-C₄hydroxyalkyl; wherein the tertiary amine oxide functions as a preservative or enhances the preservative efficacy of the pharmaceutical formulation; a pharmaceutical active agent; and sorbic acid as an additional preservative component.

2. The formulation of claim 1, wherein the tertiary amine oxide is myristamine oxide or lauramine oxide.

3. The formulation of any one of claims 1 to 2, further comprising a comfort component selected from the group consisting of hydroxypropyl guar, hyaluronic acid, alginate, dexpanthenol and hydroxypropylmethyl cellulose.

4. The formulation of claims 1 or 3, wherein the tertiary amine oxide is present from 0.5 ppm to 60 ppm.

5. The formulation of any one of claims 1 to 4, wherein the pharmaceutical agent is a prescription pharmaceutical agent.

6. The formulation of claim 5, wherein the prescription pharmaceutical agent provides for the treatment of an ocular condition or an ocular disease.

7. The formulation of any one of claims 1 to 6, wherein the formulation is a preserved ophthalmic composition having an osmolality of 225 mOsm/kg to 400 mOsm/kg.

8. The use of a tertiary amine oxide of general formula I to enhance the preservation of a topical pharmaceutical formulation that includes a pharmaceutical agent and an additional preservative component, 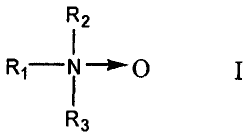 wherein R₁ is a C₈-C₁₈alkyl, and R₂ and R₃ are independently selected from a C₁-C₄alkyl or C₁-C₄hydroxyalkyl, the tertiary amine oxide is present from 0.5 ppm to 1000 ppm, and the additional preservative component is sorbic acid.

9. The use of claim 8, wherein the tertiary amine oxide is myristamine oxide or lauramine oxide.

## Patentansprüche

1. Eine topische pharmazeutische Formulierung umfassend:
0.5 ppm bis 1000 ppm eines tertiären Aminoxids der allgemeinen Formel I 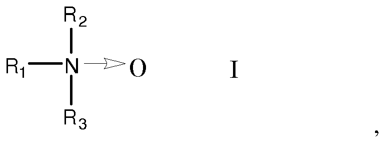
wobei R₁ ein C₈-C₁₈-Alkylrest, und R₂ und R₃ unabhängig ausgewählte C₁-C₄-Alkylreste oder C₁-C₄-Hydroxyalkylreste sind; wobei das tertiäre Aminoxid als Konservierungsmittel wirkt oder die konservierende Wirkung der pharmazeutischen Formulierung verbessert; einen pharmazeutischen Wirkstoff; und Sorbinsäure als eine zusätzliche konservierende Komponente.

2. Die Formulierung aus Anspruch 1, wobei das tertiäre Aminoxid Myristaminoxid oder Lauraminoxid ist.

3. Die Formulierung aus einem der Ansprüche 1 bis 2, weiterhin umfassend eine Komfortkomponente, ausgewählt aus der Gruppe bestehend aus Hydroxypropylguar, Hyaluronsäure, Alginat, Dexpanthenol und Hydroxypropylmethylcellulose.

4. Die Formulierung der Ansprüche 1 oder 3, wobei das tertiäre Aminoxid von 0.5 ppm bis 60 ppm enthalten ist.

5. Die Formulierung gemäß einem der Ansprüche 1 bis 4, wobei der pharmazeutische Wirkstoff ein verschreibungspflichtiger pharmazeutischer Wirkstoff ist.

6. Die Formulierung gemäß Anspruch 5, wobei der verschreibungspflichtige pharmazeutische Wirkstoff zur Behandlung eines Augenleidens oder einer Augenkrankheit dient.

7. Die Formulierung gemäß einem der Ansprüche 1 bis 6, wobei die Formulierung eine konservierte ophthalmische Zusammensetzung ist, die eine Osmolalität von 225 mOsm/kg bis 400 mOsm/kg aufweist.

8. Die Verwendung eines tertiären Aminoxids der allgemeinen Formel I zur Verbesserung der Konservierung einer topischen pharmazeutischen Formulierung, die einen pharmazeutischen Wirkstoff und eine zusätzliche konservierende Komponente beinhaltet 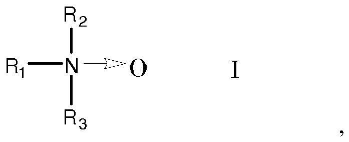 wobei R₁ ein C₈-C₁₈-Alkylrest, und R₂ und R₃ unabhängig ausgewählte C₁-C₄-Alkylreste oder C₁-C₄-Hydroxyalkylreste sind, das tertiäre Aminoxid von 0.5 ppm bis 1000 ppm enthalten ist, und die zusätzliche konservierende Komponente Sorbinsäure ist.

9. Die Verwendung gemäß Anspruch 8, wobei das tertiäre Aminoxid Myristaminoxid oder Lauraminoxid ist.

## Revendications

1. Formulation pharmaceutique topique comprenant :
0,5 ppm à 1000 ppm d'un oxyde d'amine tertiaire de formule générale I 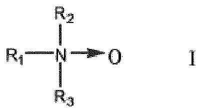
dans laquelle R₁ est un alkyle en C₈ - C₁₈, et R₂ et R₃ sont indépendamment sélectionnés parmi un alkyle en C₁ - C₄ ou un hydroxyalkyle en C₁ - C₄ ; dans laquelle l'oxyde d'amine tertiaire fonctionne comme un préservateur ou améliore l'efficacité de préservation de la formulation pharmaceutique ; un agent actif pharmaceutique ; et de l'acide sorbique comme un composant préservateur supplémentaire.

2. Formulation selon la revendication 1, dans laquelle l'oxyde d'amine tertiaire est de l'oxyde de myristamine ou de l'oxyde de lauramine.

3. Formulation selon l'une quelconque des revendications 1 à 2, comprenant en outre un composant de confort sélectionné parmi le groupe constitué du guar hydroxypropylé, d'acide hyaluronique, d'alginate, de dexpanthénol et d'hydroxypropylméthylcellulose.

4. Formulation selon la revendication 1 ou la revendication 3, dans laquelle l'oxyde d'amine tertiaire est présente de 0,5 ppm à 60 ppm.

5. Formulation selon l'une quelconque des revendications 1 à 4, dans laquelle l'agent pharmaceutique est un agent pharmaceutique délivré sur ordonnance.

6. Formulation selon la revendication 5, dans laquelle l'agent pharmaceutique délivré sur ordonnance fournit un traitement pour une condition oculaire ou une maladie oculaire.

7. Formulation selon l'une quelconque des revendications 1 à 6, dans laquelle la formulation est une composition ophtalmique préservée possédant une osmolalité de 225 mOsm/kg à 400 mOsm/kg.

8. Utilisation d'un oxyde d'amine tertiaire de formule générale I pour améliorer la préservation d'une formulation pharmaceutique topique qui inclut un agent pharmaceutique et un composant préservateur supplémentaire, 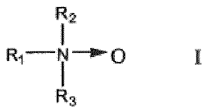 dans laquelle R₁ est un alkyle en C₈ - C₁₈, et R₂ et R₃ sont indépendamment sélectionnés parmi un alkyle en C₁₋C₄ ou un hydroxyalkyle en C₁₋C₄, l'oxyde d'amine tertiaire est présent de 0,5 ppm à 1000 ppm, et le composant préservateur supplémentaire est de l'acide sorbique.

9. Utilisation selon la revendication 8, dans laquelle l'oxyde d'amine tertiaire est de l'oxyde de myristamine ou de l'oxyde de lauramine.
